# EUROPEAN PATENT APPLICATION

(11) **EP 0 758 639 A1**
(43) Date of publication of application: **19.02.1997**
(21) Application number: 96401733.9
(22) Date of filing: 05.08.1996
(51) Int. Cl.: C07C 45/67, C07C 47/575

(54) **Process for preparing isovanillin**

(30) Priority: 11.08.1995 JP 205725/95; 05.04.1996 JP 83865/96
(71) Applicant: UBE INDUSTRIES, LTD., Ube-shi, Yamaguchi-ken 755 (JP)
(72) Inventor: Kuroda, Nobuyuki, c/o Ube Res. Lab., Ube-shi, Yamaguchi-ken (JP)
(74) Representative: Desaix, Anne

(57) **Abstract**

Disclosed is a process for preparing isovanillin, which comprises reacting a 3-alkoxy-4-methoxybenzaldehyde represented by the formula (1) : 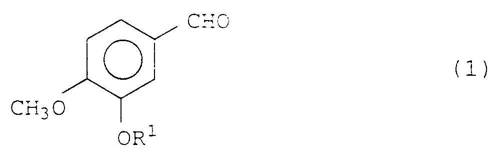 wherein R¹ represents an alkyl group or a cycloalkyl group,
with a sulfide compound represented by the formula (2) :

R²-S-R³ (2)

wherein R² represents an alkyl group or a group of the formula (3) :

-CH₂COOR⁴ (3)

wherein R⁴ represents a hydrogen atom or an alkyl group,
and R³ represents a group of the formula (4) : 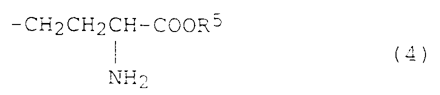 wherein R⁵ represents a hydrogen atom or an alkyl group,
or a group of the above formula (3), or a salt thereof in the presence of sulfuric acid.

## Description

### BACKGROUND OF THE INVENTION

This invention relates to a novel process for preparing isovanillin (3-hydroxy-4-methoxybenzaldehyde).

The above isovanillin is useful as an intermediate, for example, when a medicine, particularly an antidepressant is synthesized. Isovanillin can be converted into diethyl 3-isobutoxy-4-methoxybenzalmalonate according to, for example, the method described in U.S. Patent No. 4,193,926 by reacting it with isobutyl bromide to convert into 3-isobutoxy-4-methoxybenzaldehyde, and then, reacting the obtained compound with diethyl malonate. The obtained diethyl 3-isobutoxy-4-methoxybenzalmalonate is reacted with potassium cyanide to be converted into ethyl 3-(3-isobuzoxy-4-methoxyphenyl)-3-cyanopropionate, and the obtained compound is further hydrogenated in glacial acetic acid in the presence of platinum oxide and then reacted with methanolic hydrochloric acid to be converted into ethyl 3-(3-isobutoxy-4-methoxyphenyl)-4-aminobutanoate hydrochloride. The obtained ethyl 3-(3-isobutoxy-4-methoxyphenyl)-4-aminobutanoate hydrochloride can be converted into 4-(3-isobutoxy-4-methoxyphenyl)-2-pyrrolidone having an antidepressant activity by reacting it with triethyl-amine.

As a conventional process for preparing isovanillin, there may be mentioned the following processes.
(1) In Journal of Organic Chemistry (J. Org. Chem.), Vol. 32, No. 4, pp. 1269 and 1270 (1967), there has been disclosed a process for preparing isovanillin by heating veratraldehyde in conc. sulfuric acid.
   However, the above process (1) is not an industrially satisfactory process in the points that the process requires a long period of time as long as 20 hours with a low yield.
(2) In Journal of Chemical Society (J. Chem. Soc.), Perkin Transactions, Vol. 1, No. 20, pp. 2288 and 2289 (1977), there has been disclosed a process for preparing isovanillin by reacting veratraldehyde with methionine in the presence of methanesulfonic acid.

However, the above process (2) is not an industrially satisfactory process in the points that the process requires a long period of time as long as 24 hours with a low yield.

Therefore, both of the known preparation processes (1) and (2) are not satisfactory as a process for obtaining isovanillin.

### SUMMARY OF THE INVENTION

The present inventors have studied intensively in order to solve the problems in the above known preparation processes, and consequently found that when a 3-alkoxy-4-methoxybenzaldehyde is reacted with a sulfide compound in the presence of sulfuric acid, isovanillin can be obtained with a good yield without requiring a long period of time as described above, to accomplish the present invention.

An object of the present invention is to provide a process for preparing isovanillin in a short period of time with a good yield, by reacting a 3-alkoxy-4-methoxybenzaldehyde with a sulfide compound in the presence of sulfuric acid.

That is, the present invention relates to a process for preparing isovanillin, which comprises reacting a 3-alkoxy-4-methoxybenzaldehyde represented by the formula (1): 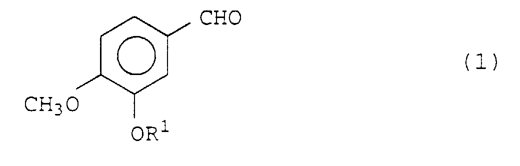 wherein R¹ represents an alkyl group or a cycloalkyl group,
with a sulfide compound represented by the formula (2):

R²-S-R³ (2)

wherein R² represents an alkyl group or a group of the formula (3):

-CH₂COOR⁴ (3)

wherein R⁴ represents a hydrogen atom or an alkyl group,
and R³ represents a group represented by the formula (4): 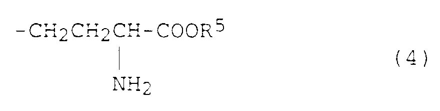 wherein R⁵ represents a hydrogen atom or an alkyl group,
or a group of the above formula (3),
or a salt thereof in the presence of sulfuric acid to obtain isovanillin (3-hydroxy-4-methoxybenzaldehyde) .

Preferred embodiments of the above preparation process of the present invention are as described below.
1) In the above process for preparing isovanillin, the 3-alkoxy-4-methoxybenzaldehyde represented by the above formula (1) is veratraldehyde.
2) In the above process for preparing isovanillin, the sulfide compound represented by the above formula (2) or a salt thereof is a methionine derivative.
3) In the above process for preparing isovanillin, the sulfide compound represented by the above formula (2) or a salt thereof is a thioglycolic acid derivative.
4) In the above process for preparing isovanillin, the sulfide compound represented by the above formula (2) or a salt thereof is methionine or methionine methyl ester.
5) In the above process for preparing isovanillin, the sulfide compound represented by the above formula (2) or a salt thereof is thiodiglycolic acid.
6 ) In the above process for preparing isovanillin, the 3-alkoxy-4-methoxybenzaldehyde represented by the above formula (1) is veratraldehyde, and the sulfide compound represented by the above formula (2) or a salt thereof is methionine or methionine methyl ester.
7) In the above process for preparing isovanillin, the 3-alkoxy-4-methoxybenzaldehyde represented by the above formula (1) is veratraldehyde, and the sulfide compound represented by the above formula (2) or a salt thereof is thiodiglycolic acid.

### DESCRIPTION OF THE PREFERRED EMBODIMENTS

In the following, the present invention is explained in detail.

The preparation process of the present invention can be represented by, for example, Reaction scheme (1) as shown below. 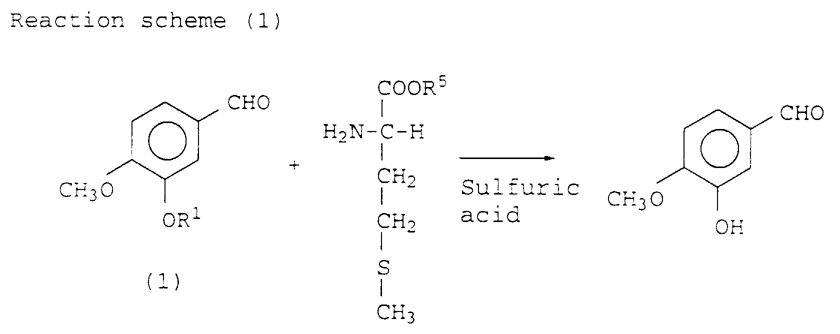 wherein R¹ and R⁵ have the same meanings as described above.

R¹ in the 3-alkoxy-4-methoxybenzaldehyde (hereinafter also referred to as "the compound (1) ") represented by the formula (1) to be used in the preparation process of the present invention represents an alkyl group or a cycloalkyl group.

As the alkyl group represented by R¹ in the compound (1), there may be mentioned, for example, an alkyl group having 1 to 10 carbon atoms, preferably a methyl group, an ethyl group, a propyl group (including the respective isomers such as n- and isopropyl groups) and a butyl group (including the respective isomers such as n-, iso-, sec- and tert-butyl groups) .

As the cycloalkyl group represented by R¹ in the compound (1), there may be mentioned, for example, a cycloalkyl group having 3 to 10 carbon atoms, preferably a cyclopropyl group, a cyclopentyl group and a cyclohexyl group.

As a specific example of the 3-alkoxy-4-methoxybenzaldehyde represented by the formula (1) having such a substituent, there may be mentioned, for example, veratraldehyde (3,4-dimethoxybenzaldehyde), 3-ethoxy-4-methoxybenzaldehyde, 3-n-butoxy-4-methoxybenzaldehyde, 3-cyclopentyloxy-4-methoxybenzaldehyde and 3-cyclohexyloxy-4-methoxybenzaldehyde, preferably veratraldehyde and 3-ethoxy-4-methoxybenzaldehyde.

R² of the sulfide compound (hereinafter also referred to as "the compound (2)") represented by the formula (2) to be used in the preparation process of the present invention represents an alkyl group or a group represented by the formula (3) .

As the alkyl group represented by R² in the compound (2), there may be mentioned, for example, an alkyl group having 1 to 10 carbon atoms, preferably a methyl group, an ethyl group, a propyl group (including the respective isomers as mentioned above) and a butyl group (including the respective isomers as mentioned above).

R⁴ of the formula (3) represented by R² in the compound (2) represents a hydrogen atom or an alkyl group.

As the alkyl group represented by R⁴ in the compound (1), there may be mentioned, for example, an alkyl group having 1 to 10 carbon atoms, preferably a methyl group, an ethyl group, a propyl group (including the respective isomers as mentioned above) and a butyl group (including the respective isomers as mentioned above) .

R³ in the compound (2) represents a group represented by the formula (3) or the formula (4).

R⁴ of the formula (3) represented by R³ in the compound (2) represents a hydrogen atom or an alkyl group.

As the alkyl group represented by R⁴ in the compound (2), there may be mentioned, for example, an alkyl group having 1 to 10 carbon atoms, preferably a methyl group, an ethyl group, a propyl group (including the respective isomers as mentioned above) and a butyl group (including the respective isomers as mentioned above).

R⁵ of the formula (4) represented by R³ in the compound (2) represents a hydrogen atom or an alkyl group.

As the alkyl group represented by R⁵ in the compound (2), there may be mentioned, for example, an alkyl group having 1 to 10 carbon atoms, preferably a methyl group, an ethyl group, a propyl group (including the respective isomers as mentioned above) and a butyl group (including the respective isomers as mentioned above).

As a specific example of the sulfide compound represented by the formula (2) having such substituents, there may be mentioned, for example, methionine derivatives such as methionine, methionine methyl ester, methionine ethyl ester, methionine propyl ester (including the respective isomers) , methionine butyl ester (including the respective isomers), methionine pentyl ester (including the respective isomers), methionine hexyl ester (including the respective isomers), methionine heptyl ester (including the respective isomers), methionine octyl ester (including the respective isomers), methionine nonyl ester (including the respective isomers) and methionine decyl ester (including the respective isomers), and thioglycolic acid derivatives such as thiodiglycolic acid, dimethyl thiodiglycolate, S-methyl-thioglycolic acid and methyl S-methylthioglycolate, preferably methionine, methionine methyl ester, methionine ethyl ester, thiodiglycolic acid and S-methylthioglycolic acid.

Among the sulfide compounds to be used in the preparation process of the present invention, the methionine derivatives may be used in any form, but the form of an inorganic acid salt such as sulfate, hydrochloride and phosphate is generally preferred. In the methionine derivatives to be used in the preparation process of the present invention, optically active D- and L-isomers exist. Either of the optically active isomers and also the racemic modification may be used in the preparation process of the present invention.

The amount of the sulfide compound to be used in the preparation process of the present invention is generally an amount of 0.5 to 3.0 mole, preferably 0.8 to 2.0 mole per 1 mole of the 3-alkoxy-4-methoxybenzaldehyde.

The concentration of sulfuric acid to be used in the preparation process of the present invention is generally 80 % by weight or more, preferably 90 % by weight or more.

The amount of sulfuric acid to be used in the preparation process of the present invention is generally in the range of 3 to 30 times, preferably 4 to 20 times the weight of the 3-alkoxy-4-methoxybenzaldehyde. If the amount of sulfuric acid to be used is less than the required amount, undesirable by-products such as vanillin are increased, while if said amount is more than the required amount, productivity is worsened.

The reaction temperature in the preparation process of the present invention is generally 20 °C to 150 °C, preferably 40 °C to 120 °C.

In the preparation process of the present invention, the reaction time may vary depending on the amount of sulfuric acid and the reaction temperature to be used, but it is preferably 0.5 to 8 hours.

The reaction can be carried out generally under atmospheric pressure, but may be carried out under pressure or under reduced pressure.

In the preparation process of the present invention, produced isovanillin can be obtained from a reaction mixture by a combination of conventional washing operation and separation operation. For example, the desired compound can be obtained by a method of adding water to the reaction mixture, stirring the mixture, subjecting the mixture to extraction with an appropriate solvent, washing with water, etc. and drying operations and carrying out recrystallization.

According to the present invention, by reacting the 3-alkoxy-4-methoxybenzaldehyde represented by the above formula (1) with the sulfide compound of the formula (2) in the presence of sulfuric acid, isovanillin which is a desired compound can be obtained in a short period of time with a good yield.

### EXAMPLES

The present invention is described in detail by referring to Examples, but the scope of the present invention is not limited thereby. Analysis conditions of liquid chromatography (analysis of veratraldehyde) and gas chromatography (analysis of isovanillin) in Examples and Comparative examples are as shown below.

### Analysis conditions of liquid chromatography (analysis of veratraldehyde)

Column: ODS-80TM (trade name, manufactured by TOSOH Co., 4.6 mm ID x 25 cm L)
Temperature: 40 °C
Eluent: H₂O : CH₃CN = 6 : 4, pH = 3.0
Flow rate: 1.0 ml/min
Detection wavelength: 254 nm
Internal standard reference material: anisole

### Analysis conditions of gas chromatography (analysis of isovanillin)

Column: 2 % Silicone OV-17 (trade name, manufactured by GL Science Co.), support: Uniport HS (trade name, manufactured by GL Science Co.)
Temperature of column : 120 to 160 °C (3 °C/min)
Temperature of evaporation room: 200 °C
Carrier gas: helium
Flow rate: 40 ml/min
Detector: FID
Internal standard reference material: n-tetradecane

### Example 1

A flask having an inner volume of 100 ml was charged with 30 g of conc. sulfuric acid (98 %), and 3.32 g (20 mmol) of veratraldehyde was added thereto while stirring to obtain a sulfuric acid solution. While stirring the obtained sulfuric acid solution at room temperature (20 °C), 3.3 g (22 mmol) of D,L-methionine was gradually added to the solution with divided small amounts over 10 minutes. After completion of the addition, the mixture was reacted under stirring at 65 °C for 3 hours.

The resulting reaction mixture was poured into 50 ml of ice water, 25 ml of ethyl acetate was added thereto, and the mixture was stirred and then separated to obtain an ethyl acetate layer (x 3 times). The combined ethyl acetate layer was quantitated by liquid chromatography and gas chromatography. As a result, the conversion ratio of veratraldehyde was 95.3 %, and the yield of isovanillin based on veratraldehyde was 82.7 %. After the obtained ethyl acetate layer was concentrated by a rotary evaporator, toluene was added to the concentrate. The mixture was heated and then cooled to room temperature to precipitate crystals, and the precipitated crystals were collected by filtration. The obtained crystals were analyzed by a mass spectrometer to find out that the molecular weight of the crystals was coincident with that of isovanillin. m/e = 18, 81, 151, 152. The isolation yield of isovanillin based on veratraldehyde was 72 %.

### Comparative example 1

A flask having an inner volume of 100 ml was charged with 30 g of conc. sulfuric acid (98 %), and 3.32 g (20 mmol) of veratraldehyde was added thereto while stirring to obtain a sulfuric acid solution. The obtained sulfuric acid solution was reacted under stirring at 65 °C for 3 hours.

The resulting reaction mixture was poured into 50 ml of ice water, 25 ml of ethyl acetate was added thereto, and the mixture was stirred and then separated to obtain an ethyl acetate layer (x 3 times). The combined ethyl acetate layer was quantitated by liquid chromatography and gas chromatography. As a result, the conversion ratio of veratraldehyde was 52.0 %, and the yield of isovanillin based on veratraldehyde was 44.1 %.

### Example 2

A flask having an inner volume of 100 ml was charged with 30 g of conc. sulfuric acid (98 %), and 3.32 g (20 mmol) of veratraldehyde was added thereto while stirring to obtain a sulfuric acid solution. While stirring the obtained sulfuric acid solution at room temperature (20 °C), 3.3 g (22 mmol) of D,L-methionine was gradually added to the solution with divided small amounts over 10 minutes. After completion of the addition, the mixture was reacted under stirring at 50 °C for 5 hours.

The resulting reaction mixture was poured into 50 ml of ice water, 25 ml of ethyl acetate was added thereto, and the mixture was stirred and then separated to obtain an ethyl acetate layer (x 3 times). The combined ethyl acetate layer was quantitated by liquid chromatography and gas chromatography. As a result, the conversion ratio of veratraldehyde was 89.0 %, and the yield of isovanillin based on veratraldehyde was 79.6 %.

### Comparative example 2

A flask having an inner volume of 100 ml was charged with 26 g of methanesulfonic acid, and 3.32 g (20 mmol) of veratraldehyde was added thereto while stirring to obtain a methanesulfonic acid solution. After the obtained methane-sulfonic acid solution was cooled with ice, 3.3 g (22 mmol) of D,L-methionine was gradually added to the solution over 10 minutes. After completion of the addition, the mixture was reacted under stirring at 50 °C for 6 hours.

The resulting reaction mixture was poured into 50 ml of ice water, 25 ml of ethyl acetate was added thereto, and the mixture was stirred and then separated to obtain an ethyl acetate layer (x 3 times). The combined ethyl acetate layer was quantitated by liquid chromatography and gas chromatography. As a result, the conversion ratio of veratraldehyde was 67.1 %, and the yield of isovanillin based on veratraldehyde was 48.8 %.

### Example 3

Procedures were carried out in the same manner as in Example 2 except for changing the reaction temperature from 50 °C to 80 °C and the reaction time from 5 hours to 1 hour. The conversion ratio of veratraldehyde was 95.9 %, and the yield of isovanillin based on veratraldehyde was 82.4 %.

### Example 4

Procedures were carried out in the same manner as in Example 3 except for changing the amount of conc. sulfuric acid used from 30 g to 20 g and the reaction time from 1 hour to 4 hours. The conversion ratio of veratraldehyde was 94.1 %, and the yield of isovanillin based on veratraldehyde was 73.8 %.

### Example 5

A flask having an inner volume of 100 ml was charged with 15 g of conc. sulfuric acid, and 2.2 g (11 mmol) of L-methionine methyl ester hydrochloride was gradually added thereto over 10 minutes while stirring under ice cooling to obtain a sulfuric acid solution. 1.66 g (10 mmol) of veratraldehyde was added to the obtained sulfuric acid solution. After completion of the addition, the mixture was reacted by stirring at 65 °C for 3 hours.

The resulting reaction mixture was poured into 40 ml of ice water, 25 ml of ethyl acetate was added thereto, and the mixture was stirred and then separated to obtain an ethyl acetate layer (x 3 times). The combined ethyl acetate layer was quantitated by liquid chromatography and gas chromatography. As a result, the conversion ratio of veratraldehyde was 75.4 %, and the yield of isovanillin based on veratraldehyde was 64.7 %.

### Example 6

Procedures were carried out in the same manner as in Example 5 except for using 1.65 g of thiodiglycolic acid in place of L-methionine methyl ester hydrochloride and changing the reaction temperature from 65 °C to 70 °C. The conversion ratio of veratraldehyde was 90.4 %, and the yield of isovanillin based on veratraldehyde was 75.1 %.

## Claims

1. A process for preparing isovanillin, which comprises reacting a 3-alkoxy-4-methoxybenzaldehyde represented by the formula (1): 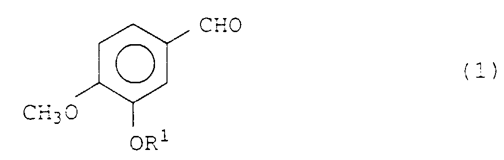 wherein R¹ represents an alkyl group or a cycloalkyl group,
with a sulfide compound represented by the formula (2):
R²-S-R³ (2)
wherein R² represents an alkyl group or a group of the formula (3):
-CH₂COOR⁴ (3)
wherein R⁴ represents a hydrogen atom or an alkyl group,
and R³ represents a group of the formula (4): 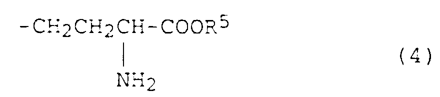 wherein R⁵ represents a hydrogen atom or an alkyl group,
or a group of the above formula (3),
or a salt thereof in the presence of sulfuric acid.

2. The process according to Claim 1, wherein R¹ is an alkyl group having 1 to 10 carbon atoms or a cycloalkyl group having 3 to 10 carbon atoms.

3. The process according to Claim 1, wherein R¹ is an alkyl group having 1 to 6 carbon atoms or a cycloalkyl group having 3 to 6 carbon atoms.

4. The process according to any one of Claims 1 to 3, wherein R¹ is a methyl group, an ethyl group, a propyl group, a butyl group, a cyclopropyl group, a cyclopentyl group or a cyclohexyl group.

5. The process according to Claim 1, wherein R¹ is a methyl group.

6. The process according to any one of Claims 1 to 5, wherein R² is an alkyl group having 1 to 10 carbon atoms or a group represented by the formula (3):
-CH₂COOR⁴ (3)
wherein R⁴ represents a hydrogen atom or an alkyl group having 1 to 10 carbon atoms.

7. The process according to any one of Claims 1 to 5, wherein R² is an alkyl group having 1 to 6 carbon atoms or a group represented by the formula (3):
-CH₂COOR⁴ (3)
wherein R⁴ represents a hydrogen atom or an alkyl group having 1 to 6 carbon atoms.

8. The process according to any one of Claims 1 to 7, wherein R² is a methyl group, an ethyl group, a propyl group, a butyl group, or a group represented by the formula (3):
-CH₂COOR⁴ (3)
wherein R⁴ represents a hydrogen atom, a methyl group, an ethyl group, a propyl group or a butyl group.

9. The process according to any one of Claims 1 to 8, wherein R² is a methyl group, -CH₂COOH or -CH₂COOCH₃.

10. The process according to any one of Claims 1 to 9, wherein R³ is a group represented by the formula (4): 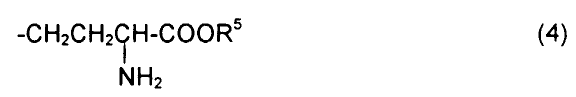 wherein R⁵ represents a hydrogen atom or an alkyl group having 1 to 10 carbon atoms,
or a group represented by the formula (3):
-CH₂COOR⁴ (3)
wherein R⁴ represents a hydrogen atom or an alkyl group having 1 to 10 carbon atoms.

11. The process according to any one of Claims 1 to 9, wherein R³ is a group represented by the formula (4): 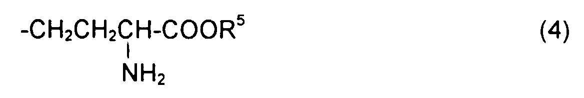 wherein R⁵ represents a hydrogen atom or an alkyl group having 1 to 6 carbon atoms,
or a group represented by the formula (3):
-CH₂COOR⁴ (3)
wherein R⁴ represents a hydrogen atom or an alkyl group having 1 to 6 carbon atoms.

12. The process according to any one of Claims 1 to 11, wherein R³ is a group represented by the formula (4): 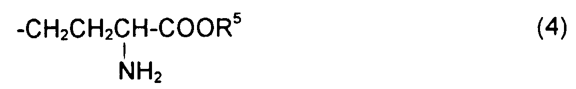 wherein R⁵ represents a hydrogen atom, a methyl group, an ethyl group, a propyl group or a butyl group,
or a group represented by the formula (3):
-CH₂COOR⁴ (3)
wherein R⁴ represents a hydrogen atom, a methyl group, an ethyl group, a propyl group or a butyl group.

13. The process according to any one of Claims 1 to 12, wherein R³ is a group represented by the formula (4): 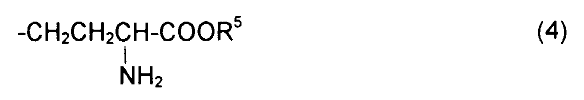 wherein R⁵ represents a hydrogen atom or a methyl group,
or a group represented by the formula (3):
-CH₂COOR⁴ (3)
wherein R⁴ represents a hydrogen atom or a methyl group.

14. The process according to any one of Claims 1 to 13, wherein R¹ is a methyl group, R² is a methyl group; and R3 is a group represented by the formula (4): 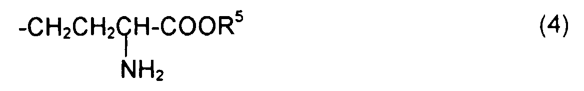 wherein R⁵ represents a hydrogen atom or a methyl group.

15. The process according to any one of Claims 1 to 10, wherein R¹ is a methyl group, R² is -CH₂COOH or -CH₂COOCH₃; and R3 is -CH₂COOH or -CH₂COOCH₃.

16. The process according to any one of Claims 1 to 15, wherein the sulfide compound is used in an amount of 0.5 to 3.0 mole per mole of the 3-alkoxy-4-methoxybenzaldehyde.

17. The process according to any one of Claims 1 to 15, wherein the sulfide compound is used in an amount of 0.8 to 2.0 mole per mole of the 3-alkoxy-4-methoxybenzaldehyde.

18. The process according to any one of Claims 1 to 15, wherein the sulfuric acid is used in an amount in the range of 3 to 30 times the weight of the 3-alkoxy-4-methoxybenzaldehyde.

19. The process according to any one of Claims 1 to 15, wherein the sulfuric acid is used in an amount in the range of 4 to 20 times the weight of the 3-alkoxy-4-methoxybenzaldehyde.

20. The process according to any one of Claims 1 to 19, wherein the reaction is carried out at 20°C to 150°C for 0.5 to 8 hours under ambient pressure.

21. The process according to any one of Claims 1 to 19, wherein the reaction is carried out at 40°C to 120°C for 0.5 to 8 hours under ambient pressure.
